# EUROPÄISCHE PATENTANMELDUNG

(11) **EP 4 108 268 A1**
(43) Veröffentlichungstag der Anmeldung: **28.12.2022**
(21) Anmeldenummer: 21180888.6
(22) Anmeldetag: 22.06.2021
(51) Int. Cl.: A61M 1/36

(54) **BIDIREKTIONALE KANÜLE**

(71) Anmelder: Salman, Jawad, 30177 Hannover (DE); Haverich, Axel, 30177 Hannover (DE)
(72) Erfinder: Salman, Jawad, 30177 Hannover (DE); Haverich, Axel, 30177 Hannover (DE)
(74) Vertreter: Scheffler, Jörg

(57) **Zusammenfassung**

Die Erfindung betrifft eine bidirektionale Kanüle (1), wobei die Kanüle (1) einen eine Kanülenwandung (2) ausbildenden Kanülenkörper (3) aufweist, welcher ein durchströmbares Hauptlumen (4) mit einer je endseitig des Kanülenkörpers (3) ausgebildeten Haupteinlassöffnung (5) und einer Hauptauslassöffnung (6) umgibt. Der Kanülenkörper (3) weist zudem ein Nebenlumen (7) mit einer Eintrittsöffnung (8) und einer Austrittsöffnung (9) auf. Hierbei sind das zumindest eine Nebenlumen (7) und das Hauptlumen (4) abgegrenzt und/oder separiert zueinander ausgebildet, sodass keine strömungsdurchlässige Verbindung zwischen Hauptlumen (4) und Nebenlumen (7) ausgebildet ist.

## Beschreibung

Die Erfindung betrifft eine bidirektionale Kanüle, wobei die Kanüle einen eine Kanülenwandung ausbildenden Kanülenkörper aufweist. Dieser Kanülenkörper umgibt ein durchströmbares Hauptlumen mit einer je endseitig des Kanülenkörpers ausgebildeten Haupteinlassöffnung und einer Hauptauslassöffnung und weist zudem ein Nebenlumen mit einer Eintrittsöffnung und einer Austrittsöffnung auf.

Die extrakorporale Membranoxygenierung (ECMO) stellt bei der intensivmedizinischen Versorgung von Patienten mit schwerem Lungen- und/oder Herzversagen eine therapeutische Maßnahme herausragender Stellung dar. Bei dieser übernimmt eine sich extrakorporal befindende Maschine zumindest teilweise die Lungen- und/oder Herzfunktion des Patienten. Man unterscheidet dabei insbesondere in veno-venöse extrakorporale Membranoxygenierung (VV-ECMO) sowie veno-arterielle extrakorporale Membranoxygenierung (VA-ECMO).

Während erstere einer geschädigten Lunge bereits die Zeit zur Heilung liefern kann, ohne dass die Lunge selbst einer Beatmung ausgesetzt wäre, eignet sich die veno-arterielle extrakorporale Membranoxygenierung zudem als Überbrückung bis zu einer Herz- und/oder Lungentransplantation, einer Kunstherztransplantation oder gar bis zur vollständigen Genesung des Patienten. Dies bedingt sich dadurch, dass bei einer VA-ECMO das nach Entnahme aus einer Vene oxygenierte Blut unter Umgehung des Herzens in eine Arterie rückgeführt wird. Eine VA-ECMO kann dabei einerseits perkutan oder aber durch eine offen chirurgische Implantation einer der Oberschenkelarterien etabliert werden.

Wird zum Anschluss einer solchen extrakorporalen Membranoxygenierung oder auch einer Herz-Lungen-Maschine eine Kanülierung der Arteria femoralis, also der Oberschenkelarterie, vorgenommen, so führen insbesondere länger andauernde Anwendungen aufgrund der im Wesentlichen proximal gerichteten Blutrückströmung und/oder einer durch die Kanülierung selbst induzierten, nahezu vollständigen Blockade der Arteria femoralis zu einer Mangeldurchblutung des Beins. Diese Komplikation tritt insbesondere bei Vorliegen einer schmächtigen oder sklerotisch veränderten Arteria femoralis auf.

Eine eine Blockade der Arteria femoralis vermeidende Anpassung der Kanülengröße ist dabei jedoch meist nicht möglich, da ein unterer Grenzwert des Blutvolumenstroms in die Arteria femoralis gewährleistet werden muss. So ist es nach länger andauernder Mangeldurchblutung des Beins häufig notwendig, operative Revisionen an der Kanülierungsstelle durchzuführen. Im ungünstigsten Fall kann aber auch hiernach das Bein nicht erhalten werden, was folglich eine Amputation des Beins erfordert.

Um eine Mangeldurchblutung des Beins und eine damit gegebenenfalls auftretende Nekrose des Beins zu vermeiden wird zur Sicherstellung einer distal gerichteten Durchblutung des Beins neben der proximal gerichteten Hauptkanülierung regelmäßig eine zweite, distal gerichtete Nebenkanülierung der Arteria femoralis vorgesehen. Die hierfür vorgesehene Kanüle weist in der Regel einen geringeren Durchmesser auf als eine für die Hauptkanülierung verwendete Kanüle, wobei dieser für gewöhnlich Blut über einen Seitenarm der die Hauptkanülierung versorgenden Blutpumpe zugeführt wird. Als nachteilig ist hierbei einerseits die Notwendigkeit eines zweiten Zugangs für die Nebenkanülierung zu betrachten, welcher eine weitere Wunde, die der Heilung bedarf, sowie einen möglichen, zusätzlichen Eintrittsbereich für Infektionen darstellt. Darüber hinaus ist die Ausführung des Seitenarms vonnöten, wodurch zusätzliche Kosten entstehen.

Um diese Nachteile zu adressieren, sind aus dem Stand der Technik bereits Lösungen bekannt, welche die distal gerichtete Durchblutung des Beins ohne die Ausführung einer Nebenkanülierung sicherstellen sollen.

So beschreibt die WO 2012 / 135 904 A1 eine bidirektionale Kanüle zur Kanülierung der Oberschenkelarterie, über welche neben einer proximal gerichteten auch eine distal gerichtete Blutversorgung gewährleistet werden soll. Hierbei ist seitlich in der Kanülenwand der Kanüle eine Öffnung ausgebildet. Dabei ist vorgesehen, dass die Öffnung entlang der Kanüle derart positioniert ist, dass diese in einem Knickbereich der Kanüle liegt, welcher nach Einführen der Kanüle an der Eintrittsstelle der Kanüle in die Oberschenkelarterie zum Liegen kommt. Der Abschnitt des Knickbereichs, in dem die Öffnung ausgeformt ist, muss folglich vollständig in die Oberschenkelarterie eingeführt werden. Aufgrund der Positionierung der Öffnung in dem Knickbereich ermöglicht die Öffnung eine distal gerichtete Blutversorgung entgegen der Hauptströmungsrichtung der Kanüle. Dabei ist an der Kanüle rückseitig der Öffnung zudem eine radiale Ausstülpung ausgeformt, um das Einführen und Positionieren der Kanüle und insbesondere das Positionieren der Öffnung in der Oberschenkelarterie zu erleichtern. Zudem soll durch die Ausstülpung ein Freibereich zwischen Arterienwand und Öffnung bereitgestellt werden, um einen Verschluss der Öffnung zu vermeiden.

Diese Lösung sowie auch die Ausführung einer Nebenkanülierung weisen den Nachteil auf, dass eine stets sichere Lage der Öffnung sowie der Nebenkanülierung in Richtung der distalen Oberschenkelarterie nicht gewährleistet werden kann, wobei jede Mobilisation eines Patienten die ordnungsgemäße Lage gefährdet. Dies kann wiederum zu einer Mangeldurchblutung des Beins führen. Gerade in Notfallsituationen, wie z. B. einer notwendigen Reanimation eines Patienten, stellt bereits das Etablieren der Hauptkanülierung eine große Herausforderung dar, wobei das primäre Ziel in der möglichst kurzfristigen Wiederherstellung des Kreislaufs des Patienten besteht. Die Kontrolle der ordnungsgemäßen Lage der Öffnung stellt sich somit schwierig dar.

Bezüglich der Nebenkanülierung kann sich zudem der Versuch deren Etablierung bei bereits eingerichteter Hauptkanülierung schwierig gestalten. Es besteht hierbei die Gefahr mehrerer Fehlpunktionen, welche insbesondere bei einer aufgrund eines Kreislaufstillstands entgleisten Gerinnung oder der Wirkung einiger Notfallmedikamente wie Gerinnungshemmern zur Entstehung von Blutungen und Hämatomen bei zusätzlich weiterhin drohender Mangeldurchblutung des Beins führen können. Darüber hinaus stellt auch die Explantation respektive die Nachsorge nach Explantation der Haupt- und Nebenkanülierung eine Herausforderung dar. Da im Falle einer perkutanen Implantation von Haupt- und Nebenkanülierung zwei Punktionsstellen vorliegen, kann eine exakte Platzierung der in der Regel zur Nachsorge verwendeten Femoralkompressionssysteme nicht stets gewährleistet werden. Dies kann folglich zu unerwünschten Nachblutungen führen.

Durch den Stand der Technik werden zudem weitere Lösungen offenbart, welche jedoch geeignet sein können, diese Problematik zu minimieren.

So ist aus der US 5 171 218 A eine weitere bidirektionale Kanüle zur Kanülierung der Oberschenkelarterie bekannt, welche eine seitliche Umlenköffnung in der Kanülenwand aufweist, sodass den Kanülenkanal durchströmendes Blut seitlich der Kanüle austreten kann. Um einen Verschluss der Umlenköffnung durch eine Anlage der Umlenköffnung an der Arterienwand zu vermeiden, sind seitlich der Umlenköffnung zwei Stützdorne ausgeformt, über welche ein Freibereich zwischen Arterienwand und Umlenköffnung vorgehalten werden soll.

Auch der US 2018 / 0 043 085 A1 lässt sich eine bidirektionale Kanüle zur Kanülierung der Oberschenkelarterie entnehmen. Hierbei werden zwei Ausführungsformen der Kanüle offenbart, wobei die Kanüle in beiden Ausführungsformen im Grunde eine seitlich in der Kanüle ausgebildete Öffnung aufweist. In erster Ausführungsform ist die Öffnung zwischen einem zylindrischen Kanülenabschnitt und einem Kanülenabschnitt, welcher radial eine U-förmige Einbuchtung aufweist, ausgebildet. Die Einbuchtung bildet dabei eine Passage, über welche ein Freibereich zwischen Arterienwand und Umlenköffnung gewährleistet werden soll. In zweiter Ausführungsform weist die Kanüle hingegen lediglich einen zylindrisch ausgeformten Abschnitt auf, wobei die seitlich in der Kanüle ausgebildete Öffnung von einer einseitig, entgegen der Hauptströmungsrichtung des Kanülenkanals geöffneten Leitabdeckung umhüllt wird. Die Leitabdeckung ist dabei in Umfangsrichtung lediglich in dem Abschnitt der Kanüle ausgeführt, welcher den Bereich der Öffnung umgibt. Beide Ausgestaltungen sollen hierbei eine distal gerichtete Blutversorgung gewährleisten.

Durch die WO 2019 / 075 020 A1 wird eine zu einer Ausführungsform der US 2018 / 0 043 085 A1 vergleichbare bidirektionale Kanüle zur Kanülierung der Oberschenkelarterie beschrieben, wobei die Kanüle zylindrisch ausgeformt und seitlich in der Kanülenwand der Kanüle eine Öffnung ausgebildet ist. Die Öffnung wird dabei von einer einseitig entgegen der Hauptströmungsrichtung des Kanülenkanals geöffneten Leitabdeckung umhüllt. Die Leitabdeckung ist dabei in Umfangsrichtung lediglich in dem Abschnitt der Kanüle ausgeführt, welcher den Bereich der Öffnung umgibt.

Der WO 2020 / 225 034 A1 lässt sich eine wiederum zur US 2018 / 0 043 085 A1 und WO 2019 / 075 020 A1 gleichartig ausgeführte bidirektionale Kanüle zur Kanülierung der Oberschenkelarterie entnehmen. Diese verfügt dabei über einen Umlenkkörper, welcher zumindest eine seitlich in der Kanülenwand der Kanüle ausgebildete Öffnung umhüllt und einseitig, entgegen der Hauptströmungsrichtung des Kanülenkanals geöffnet ausgeführt ist oder weitere Austrittsöffnungen aufweist, sodass eine distal gerichtete Blutversorgung gewährleistet werden kann. Der Umlenkkörper ist dabei in Umfangsrichtung der Kanüle vollständig umlaufend ausgeführt und dabei kappen- oder ballonförmig ausgebildet. Zur Überprüfung des aus dem Seitenloch austretenden Blutstroms weist die Kanüle einen innen an der Kanülenwand angeordneten Druckmessschlauch auf.

Den dargestellten Lösungen ist es hierbei gemein, dass die zur Versorgung des Beins vorgesehene distal gerichtete Blutversorgung unmittelbar aus dem Kanülenkanal, über welchen zudem die proximal gerichtete Blutversorgung realisiert ist, durch eine seitliche Öffnung in der Kanüle in die Oberschenkelarterie austritt. Welches Blutvolumen hierbei tatsächlich austritt und somit für die Blutversorgung des Beins zur Verfügung steht, lässt sich mit einer derartigen Ausgestaltung der Kanüle jedoch nicht bestimmen, da lediglich der gesamte Blutvolumenstrom in dem Kanülenkanal bestimmbar ist. Auch die Anordnung zusätzlicher Druckaufnehmer lässt keinen Rückschluss zu, da ein in distaler Richtung entfernt zur Öffnung vorhandener Verschluss oder Teilverschluss der Oberschenkelarterie einen Blutrückstau und somit eine Druckerhöhung zur Folge hat.

Vor diesem Hintergrund liegt der Erfindung somit die Aufgabe zugrunde, eine bidirektionale Kanüle der eingangs genannten Art zur Verfügung zu stellen, mittels welcher eine distal gerichtete Blutversorgung eines Beins sichergestellt werden kann.

Diese Aufgabe wird erfindungsgemäß mit einer bidirektionalen Kanüle gemäß den Merkmalen des Anspruchs 1 gelöst. Die weitere Ausgestaltung der Erfindung ist den Unteransprüchen zu entnehmen.

Erfindungsgemäß ist also eine zum zumindest abschnittsweisen Einbringen oder Einführen in ein Blutgefäß, insbesondere eine Oberschenkelarterie, eingerichtete bidirektionale Kanüle vorgesehen, welche insbesondere bei einer extrakorporalen Membranoxygenierung Verwendung findet. Hierfür soll die Kanüle oder zumindest ein Abschnitt der Kanüle in bevorzugter Ausgestaltung eine im Wesentlichen zylindrische Form aufweisen. Es können dabei auch mehrere, insbesondere zumindest zwei zylindrisch ausgeformte Abschnitte vorgesehen sein, welche sich voneinander unterscheidende Durchmesser aufweisen. Hierdurch bestünde die Möglichkeit, einen stromauf und somit insbesondere näher an einer Blutpumpe gelegenen Abschnitt der Kanüle, welcher nicht in das Blutgefäß eingeführt wird, mit einem größeren Durchmesser auszulegen als ein Abschnitt, welcher in das Blutgefäß eingeführt und folglich an die Größe des Blutgefäßes angepasst ist. Damit ließe sich die Kanüle hinsichtlich der Handhabung und/oder der Verbindung mit weiteren Komponenten wie Schläuchen oder Kupplungen verbessern, da ein vergrößerter Greif- und/oder Anschlussbereich vorliegt. Ferner ist zudem denkbar, dass die Kanüle oder zumindest ein Abschnitt der Kanüle konisch ausgeformt ist. Weiterhin sei erwähnt, dass die Kanüle bevorzugt aus einem flexiblen Material, wie einem hochelastischen Kunststoff oder einem Gummi, und somit leicht biegbar ausgestaltet ist.

Darüber hinaus weist die Kanüle erfindungsgemäß einen eine Kanülenwandung ausbildenden Kanülenkörper auf, wobei dieser Kanülenkörper ein durchströmbares Hauptlumen - insbesondere radial - umgibt respektive dieses ausbildet. An den jeweiligen Enden des Kanülenkörpers ist dabei eine entsprechende Haupteinlassöffnung und eine Hauptauslassöffnung ausgebildet. Über die Haupteinlass- und die Hauptauslassöffnung lässt sich nunmehr eine Primärströmungsrichtung definieren, wobei diese von der Haupteinlassöffnung zur Hauptauslassöffnung gerichtet ist. Das Hauptlumen, welches auch als Hauptkanal bezeichnet werden kann und somit grundsätzlich einen Hohlraum darstellt, ist entsprechend entlang dieser Primärströmungsrichtung durchströmbar ausgebildet. Die Primärströmungsrichtung entspräche zudem der Systemflussrichtung für eine proximal gerichtete Blutrückströmung oxygenierten, also sauerstoffangereicherten Bluts in den Körper, und hierbei insbesondere in die Oberschenkelarterie eines Patienten. Diese proximal gerichtete Blutströmung entspricht hierbei im Wesentlichen einer Primärströmung durch das Hauptlumen.

Erfindungsgemäß weist der Kanülenkörper zudem ein - insbesondere durchströmbares - Nebenlumen mit einer Eintrittsöffnung und einer Austrittsöffnung auf. Über diese lässt sich folgerichtig eine Sekundärströmungsrichtung definieren, wobei die Sekundärströmungsrichtung von der Eintrittsöffnung in Richtung der Austrittöffnung gerichtet ist. Die Austrittsöffnung ist hierbei bevorzugt seitlich in der Kanülenwandung ausgeformt respektive an einer Außenmantelseite der Kanülenwandung positioniert. Das Nebenlumen, welches auch als Nebenkanal bezeichnet werden kann und somit grundsätzlich einen Hohlraum darstellt, ist entsprechend entlang dieser Sekundärströmungsrichtung durchströmbar ausgebildet. Die Sekundärströmungsrichtung, welche dabei an der Austrittsöffnung des Nebenlumens vorliegt, entspricht im Wesentlichen der Beinperfusionsrichtung für eine distal gerichtete Blutrückströmung oxygenierten Bluts in den Körper eines Patienten. Diese über das Nebenlumen distal gerichtete Blutrückströmung muss zur Blutversorgung des Beins der über das Hauptlumen proximal gerichteten Blutströmung folglich im Wesentlichen entgegengerichtet sein, wobei die distal gerichtete Blutströmung hierbei insbesondere im Hinblick auf den Blutvolumenstrom im Wesentlichen einer Sekundärströmung durch das Nebenlumen entspricht. Daraus ergibt sich, dass auch die an der Austrittsöffnung vorliegende Sekundärströmungsrichtung der austretenden Sekundärströmung der an der Hauptauslassöffnung vorliegenden Primärströmungsrichtung der austretenden Primärströmung entgegengerichtet sein muss respektive ist, wobei auch geringe Abweichungen von einem Relativwinkel zwischen Primärströmungsrichtung und Sekundärströmungsrichtung in Höhe von 180 Grad zulässig sind. Diese geringen Abweichungen können dabei einen Bereich von bis zu 20 Grad, bevorzugt bis zu 10 Grad und/oder besonders bevorzugt bis zu 5 Grad umfassen.

Das Nebenlumen weist zudem ein geringeres Volumen als das Hauptlumen auf, was sich insbesondere in einer Lumenweite und hierbei im Speziellen dem jeweiligen Lumendurchmesser von Haupt- und Nebenlumen äußert. Eine Lumenweite respektive der Lumendurchmesser des Nebenlumens ist folglich kleiner als eine Lumenweite respektive der Lumendurchmesser des Hauptlumens.

Überdies ist erfindungsgemäß vorgesehen, dass das zumindest eine Nebenlumen und das Hauptlumen abgegrenzt und/oder separiert zueinander - respektive miteinander unverbunden - ausgebildet sind, sodass - entsprechend innerhalb der Kanüle respektive des Kanülenkörpers - keine strömungsdurchlässige Verbindung zwischen Hauptlumen und Nebenlumen ausgebildet ist. Das Hauptlumen und das Nebenlumen bilden somit im Grunde gegenüber einander geschlossene Systeme, zwischen welchen kein Stofftransport und folglich keine strömungsdurchlässige Verbindung innerhalb der Kanüle ermöglicht ist. Dies birgt den Vorteil, dass Hauptlumen und Nebenlumen insbesondere über eine oder mehrere Blutpumpen mit - an einen Patienten angepassten - individuellen Blutvolumenströmen und/oder Blutdrücken beaufschlagt werden können. In diesem Zusammenhang stellt es sich zudem als gewinnbringend dar, dass der Blutvolumenstrom durch das Hauptlumen sowie das Nebenlumen ohne zusätzliche Messaufnehmer in der Kanüle separat gemessen und überwacht werden kann, sodass eine individuelle Versorgung eines Patienten verbessert wird.

In einer besonders vorteilhaften Weiterbildung der Erfindung ist das Nebenlumen zudem zumindest abschnittsweise - bevorzugt jedoch vollständig - in der Kanülenwandung ausgeformt. So lässt sich eine bidirektionale Kanüle verwirklichen, welche trotz der zumindest zwei voneinander getrennt ausgeführten Lumen, hierbei dem Haupt- und dem zumindest einem Nebenlumen, eine kompakte, insbesondere schlanke Form aufweist und sich so weitgehend optimal in ein Blutgefäß einbringen lässt. Das Nebenlumen könnte dabei über dessen gesamten Verlauf zwischen Eintrittsöffnung und Austrittsöffnung einen kreisförmigen Querschnitt aufweisen, wobei auch weitere Gestaltungen des Nebenlumens denkbar sind. So könnte das Nebenlumen einen lichten Querschnitt aufweisen, welcher sich über den Verlauf des Nebenlumens ändert, sich dabei beispielsweise von einem Querschnitt höherer Querschnittsfläche zu einem Querschnitt mit in Relation niedrigerer Querschnittsfläche verjüngt. Überdies könnte sich neben der Querschnittsfläche auch die Querschnittsform des Nebenlumens über dessen Verlauf verändern. Ferner besteht die Möglichkeit, dass das Nebenlumen derart ausgeführt ist, dass die Kanülenwandung wenigstens abschnittsweise doppelwandig ausgebildet ist, wobei die Ausbildung des Nebenlumens in der Doppelwand z. B. über mehrere zwischen der Doppelwand ausgeformte Stützen gewährleistet wird.

Eine sich von der zuvor erläuterten Weiterbildung unterscheidende, jedoch nicht minder Erfolg versprechende Ausgestaltung der Erfindung ist darin zu sehen, dass der Kanülenkörper eine an einer Innenmantelseite und/oder einer Außenmantelseite der Kanülenwandung ausgeformte, das Nebenlumen - insbesondere radial - umgebende respektive ausbildende Sekundärleitung aufweist. Dies führt insbesondere im Vergleich zu einer Ausformung des Nebenlumens in der Kanülenwandung zu einer vereinfachten Herstellung der Kanüle, wobei eine getrennte Ausführung von Hauptlumen und Nebenlumen trotzdem sichergestellt ist. Die Sekundärleitung kann einerseits vollständig an der Außenmantelseite oder aber teilweise an der Innenmantelseite und teilweise an der Außenmantelseite der Kanülenwandung ausgeformt sein, wobei die Sekundärleitung in letzterer Gestaltung von der Innenmantelseite auf die Außenmantelseite der Kanülenwandung übergeht und diese dementsprechend durchdringen würde respektive einen Teil der Kanülenwandung ausbildet.

Neben den vorstehend erläuterten Ausgestaltungen des Nebenlumens bestünde weiterhin die Möglichkeit, dass das Nebenlumen teilweise in der Kanülenwandung und teilweise über eine an der Innenmantelseite und/oder der Außenmantelseite der Kanülenwandung ausgeformte Sekundärleitung ausgeführt ist.

Eine Weiterbildung der Erfindung lässt sich zudem dann als vielversprechend betrachten, wenn die Eintrittsöffnung an der Stirnseite der Kanülenwandung positioniert ist, an welcher sich die Haupteinlassöffnung befindet. Die Haupteinlassöffnung des Hauptlumens und die Eintrittsöffnung des Nebenlumens wären hierbei parallel zueinander ausgerichtet und würden im Speziellen gar in einer Ebene liegen. Hierdurch lassen sich das Hauptlumen und das Nebenlumen aus lediglich einer Richtung, welche sich insbesondere mit der Hauptströmungsrichtung deckt, mit weiteren Komponenten wie Schläuchen, Kupplungen und/oder einer oder mehreren Blutpumpen verbinden. So könnte auch eine Ankopplung von Haupt-und Nebenlumen über eine einzige Kupplung erfolgen. Durch eine solch einseitige Anordnung werden überdies die Erreichbarkeit des über die Kanüle etablierten Zugangs zur Oberschenkelarterie und somit die Wundpflege erleichtert. Weiterhin wird die Gefahr eines unbeabsichtigten Entfernens der Kanüle, z. B. aufgrund einer versehentlichen Zugausübung auf die mit der Kanüle verbundenen Komponenten bei der Mobilisierung eines Patienten durch Pflegepersonal, minimiert. Das Risiko einer damit gegebenenfalls verbundenen Ruptur der Oberschenkelarterie ließe sich somit ebenfalls verringern.

In einer hiervon abweichenden Ausbildung der Erfindung ist hingegen vorgesehen, dass die Eintrittsöffnung an einer Außenmantelseite der Kanülenwandung positioniert ist. Grundsätzlich würde dies zwar die Erreichbarkeit des vorstehend erläuterten Zugangs erschweren, da eine Verbindung mit zusätzlichen Komponenten aus im Wesentlichen zwei Richtungen etabliert werden muss, jedoch vereinfacht dies die Beaufschlagung von Haupt- und Nebenlumen mit sich unterscheidenden Blutvolumenströmen. Dies wird insbesondere dadurch bedingt, dass eine Ankopplung von Haupt- und Nebenlumen separat beaufschlagender Blutpumpen, über welche der jeweilige Blutvolumenstrom eingestellt werden kann, deutlich vereinfacht wird. Weiterhin wird durch die Verbindung aus zwei im Wesentlichen senkrecht aufeinander stehenden Richtungen eine räumliche Entzerrung bewirkt, sodass eine Verbindung zu Haupt- und Nebenlumen bedingt durch die notwendige Größe der Kanüle sowie der für die Verbindungen genutzten Kupplungen gar erst ermöglicht wird.

Eine Erfolg versprechende Ausgestaltung der erfindungsgemäßen Kanüle zeichnet sich ferner dadurch aus, dass das Nebenlumen wenigstens einen linearen Lumenabschnitt und/oder wenigstens einen - insbesondere dem linearen Lumenabschnitt vor- und/oder nacheilenden - gekrümmten Lumenabschnitt aufweist. Diese Ausgestaltung bietet eine einfache, jedoch effektive Lösung, das Nebenlumen derart auszubilden, dass die an der Austrittsöffnung des Nebenlumens austretende Sekundärströmung der an der Hauptauslassöffnung des Hauptlumens austretenden Primärströmung entgegengerichtet ist. Im einfachsten Fall ließe sich die der Primärströmung entgegengerichtete Sekundärströmung über lediglich einen linearen Lumenabschnitt und einen gekrümmten Lumenabschnitt verwirklichen. Der gekrümmte Lumenabschnitt würde hierbei bevorzugt eine Wendung von 180 Grad vollziehen. Ist die Eintrittsöffnung hierbei stirnseitig positioniert, würde sich der lediglich eine lineare Lumenabschnitt des Nebenlumens in Richtung der Austrittsöffnung an die Eintrittsöffnung anschließen respektive dieser nacheilen. An den linearen Abschnitt würde sich in Richtung der Austrittsöffnung wiederum der lediglich eine gekrümmte Lumenabschnitt anschließen respektive diesem nacheilen, wobei der gekrümmte Abschnitt in die Austrittsöffnung des Nebenlumens mündet.

Somit ist in einer Gestaltungsform der Erfindung angedacht, dass ein der Eintrittsöffnung in Richtung der Austrittsöffnung nacheilender - und/oder ein der Austrittsöffnung in Richtung der Eintrittsöffnung voreilender - Lumenabschnitt des Nebenlumens linear ausgebildet und zum Hauptlumen oder einem Abschnitt des Hauptlumens - im Wesentlichen - parallel ausgerichtet ist. Hierdurch ließe sich das Nebenlumen bei einer bevorzugt stirnseitig positionierten Eintrittsöffnung des Nebenlumens bis in einen Abschnitt der Kanüle ausbilden, welcher sich innerhalb des Blutgefäßes befindet. Dadurch ließe sich auch der das Nebenlumen insbesondere bei Einsatz oder Verwendung der Kanüle bei einer exkorporalen Membranoxygenierung durchströmende Sekundärstrom zunächst bis in diesen sich innerhalb des Blutgefäßes befindenden Abschnitt der Kanüle überführen.

Eine allgemeine, jedoch insbesondere mit der Ausgestaltung eines linearen Lumenabschnitts des Nebenlumens zusammenhängende Weiterbildung der erfindungsgemäßen Kanüle lässt sich dadurch als vorteilhaft ansehen, dass ein der Austrittsöffnung in Richtung der Eintrittsöffnung voreilender - oder ein der Eintrittsöffnung in Richtung der Austrittsöffnung nacheilender - Lumenabschnitt des Nebenlumens gekrümmt ausgebildet ist. Der gekrümmte Abschnitt folgt hierbei einem Bogen, insbesondere einem Kreisbogen, wobei eine in der Austrittsöffnung - oder der Eintrittsöffnung - an den Bogen angelegte Tangente parallel oder in einem spitzen Winkel zum Hauptlumen ausgerichtet ist. Dies führt gewinnbringend dazu, dass ein aus der Austrittsöffnung, welche auf den derart ausgeformten gekrümmten Lumenabschnitt folgt, austretender Sekundärstrom im Wesentlichen dem aus der Hauptauslassöffnung austretenden Primärstrom entgegengerichtet ist.

Eine im Rahmen der Erfindung zudem angedachte und überaus vielversprechende Ausführungsform besteht darin, dass im Nebenlumen eine Zweitkanüle angeordnet ist, welche sich - zumindest in eingebrachtem Zustand der Kanüle - über die Austrittöffnung oder die Eintrittsöffnung und die Austrittsöffnung hinaus erstreckt. Das Nebenlumen dient demnach nicht unmittelbar zur Leitung der Sekundärströmung, sondern zur Lagerung der Zweitkanüle, welche dann entsprechend die Leitung der Sekundärströmung übernimmt. Aufgrund dessen, dass sich die Zweitkanüle wenigstens über die Austrittsöffnung und dabei soweit über die Austrittsöffnung hinaus erstreckt, sodass ein Auslass der Zweitkanüle, im in die Oberschenkelarterie eingebrachten Zustand der Kanüle distal des Zugangs zur Oberschenkelarterie positioniert ist, kann eine Blutversorgung des Beins besonders zuverlässig sichergestellt werden.

In diesem Zusammenhang stellt es sich ferner als günstig dar, wenn das Nebenlumen als Führung für die Zweitkanüle ausgebildet ist und die Zweitkanüle - somit - gegenüber dem Kanülenkörper verschiebbar im Nebenlumen angeordnet ist. Hierdurch kann einerseits das Einbringen der Kanüle in die Oberschenkelarterie vereinfacht werden, indem die Kanüle entweder zunächst ohne im Nebenlumen angeordneter Zweitkanüle in die Oberschenkelarterie eingebracht und diese nach dem Einbringen in das Nebenlumen eingeführt wird oder beim Einbringen ein Überstand der Zweitkanüle über die Austrittsöffnung des Nebenlumens vermieden wird. Andererseits lässt sich die Position der Zweitkanüle und dabei insbesondere des Auslasses der Zweitkanüle aufgrund der verschiebbaren Anordnung innerhalb des Nebenlumens individuell an einen Patienten anpassen, sodass sich die Sicherstellung der Blutversorgung des Beins weiter steigern lässt. Bei einer Entnahme der Kanüle ließe sich zudem die Gefahr einer Ruptur der Oberschenkelarterie und/oder des umliegenden Gewebes minimieren, indem zunächst die Zweitkanüle aus dem Nebenlumen und somit der Oberschenkelarterie entfernt wird.

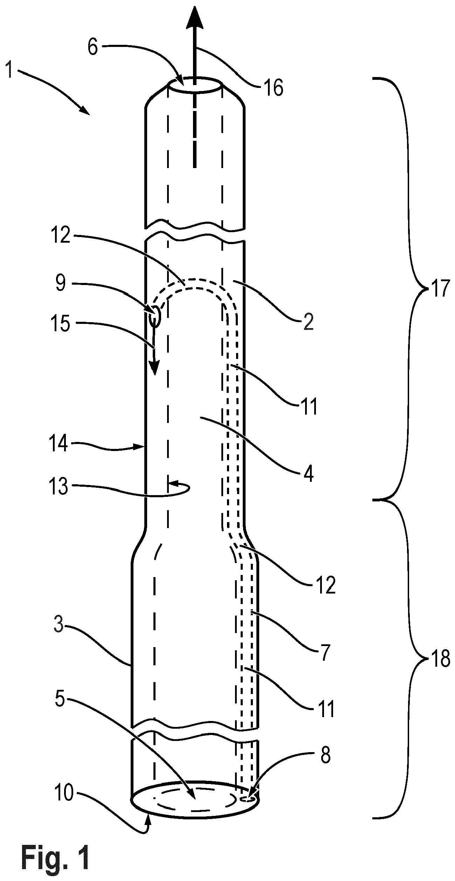

Die Erfindung lässt verschiedene Ausführungsformen zu. Zur weiteren Verdeutlichung ihres Grundprinzips ist eine davon in der Zeichnung dargestellt und wird nachfolgend beschrieben. Diese zeigt in
- Fig. 1: eine bidirektionale Kanüle mit in der Kanülenwandung ausgeformtem Nebenlumen.

Figur 1 ist dabei eine vereinfachte Darstellung einer möglichen Ausführungsform der erfindungsgemäßen bidirektionalen Kanüle 1 zu entnehmen, wobei die Kanüle 1 hierbei den die Kanülenwandung 2 ausbildenden Kanülenkörper 3 aufweist. Der Kanülenkörper 3 umgibt dabei das durchströmbare Hauptlumen 4 respektive bildet der Kanülenkörper 3 dieses Hauptlumen 4 aus. Je endseitig des Kanülenkörpers 3 sind zudem die Haupteinlassöffnung 5 und die Hauptauslassöffnung 6 ausgebildet. Neben dem Hauptlumen 4 weist der Kanülenkörper 3 zudem das in der Kanülenwandung 2 ausgeformte Nebenlumen 7 mit der Eintrittsöffnung 8 und der Austrittsöffnung 9 auf. Das Nebenlumen 7 und das durch die Innenmantelseite 13 begrenzte Hauptlumen 4 sind hierbei zueinander abgegrenzt und entsprechend separiert ausgebildet, sodass keine strömungsdurchlässige Verbindung zwischen Hauptlumen 4 und Nebenlumen 7 ausgebildet ist. Die Eintrittsöffnung 8 ist zudem an der Stirnseite 10 der Kanülenwandung 2 positioniert, an welcher sich die Haupteinlassöffnung 5 befindet. Ferner ist die Austrittsöffnung 9 in der Kanülenwandung 2 ausgeformt und dabei an der Außenmantelseite 14 der Kanülenwandung 2 positioniert. Darüber hinaus weist das Nebenlumen 7 lineare Lumenabschnitte 11 und gekrümmte Lumenabschnitte 12 auf, wobei der der Eintrittsöffnung 8 in Richtung der Austrittsöffnung 9 nacheilende respektive stromab ausgeformte Lumenabschnitt 11 des Nebenlumens 7 entsprechend linear ausgebildet, ferner zum Hauptlumen 4 parallel ausgerichtet ist und sich von der Stirnseite 10 in Richtung der Hauptauslassöffnung 6 erstreckt. Der der Austrittsöffnung 9 in Richtung der Eintrittsöffnung 8 voreilende respektive stromauf ausgeformte Lumenabschnitt 12 des Nebenlumens 7 ist hingegen gekrümmt ausgebildet und vollzieht eine Wendung von 180 Grad, sodass eine an der Austrittsöffnung 9 aus dem Nebenlumen 7 austretende Sekundärströmung eine Sekundärströmungsrichtung 15 aufweist, welche der aus dem Hauptlumen 4 in Primärströmungsrichtung 16 austretenden Primärströmung im Wesentlichen entgegengerichtet ist. Zwischen dem der Eintrittsöffnung 8 nacheilenden linearen Lumenabschnitt 11 und dem der Austrittsöffnung 9 voreilenden gekrümmten Lumenabschnitt 12 ist jeweils ein weiterer, stromab folgender gekrümmter Lumenabschnitt 12 und ein auf diesen stromab folgender linearer Lumenabschnitt 11 ausgebildet, auf den der bereits genannte, zur Austrittsöffnung 9 führende gekrümmte Lumenabschnitt 12 folgt. Dabei wird es durch die zwei linearen Lumenabschnitte 11 und den zwischen diesen liegenden gekrümmten Lumenabschnitt 12 ermöglicht, einen das Nebenlumen 7 insbesondere bei Einsatz oder Verwendung der Kanüle 1 bei einer exkorporalen Membranoxygenierung durchströmenden Sekundärstrom zunächst bis in den sich innerhalb eines Blutgefäßes befindenden Kanülenabschnitt 17 der Kanüle 1 zu überführen und diesen anschließend über den der Austrittsöffnung 9 voreilenden gekrümmten Lumenabschnitt 12 zur distalen Blutversorgung eines Beins aus der Austrittsöffnung 9 austreten zu lassen. Der Kanülenabschnitt 18 befände sich hingegen außerhalb des Blutgefäßes.

### BEZUGSZEICHENLISTE

- 1: Kanüle
- 2: Kanülenwandung
- 3: Kanülenkörper
- 4: Hauptlumen
- 5: Haupteinlassöffnung

- 6: Hauptauslassöffnung
- 7: Nebenlumen
- 8: Eintrittsöffnung
- 9: Austrittsöffnung
- 10: Stirnseite

- 11: linearer Lumenabschnitt
- 12: gekrümmter Lumenabschnitt
- 13: Innenmantelseite
- 14: Außenmantelseite
- 15: Sekundärströmungsrichtung
- 16: Primärströmungsrichtung

- 17: Kanülenabschnitt
- 18: Kanülenabschnitt

## Patentansprüche

1. Bidirektionale Kanüle (1), wobei die Kanüle (1) einen eine Kanülenwandung (2) ausbildenden Kanülenkörper (3) aufweist, welcher ein durchströmbares Hauptlumen (4) mit einer je endseitig des Kanülenkörpers (3) ausgebildeten Haupteinlassöffnung (5) und einer Hauptauslassöffnung (6) umgibt und zudem ein Nebenlumen (7) mit einer Eintrittsöffnung (8) und einer Austrittsöffnung (9) aufweist, **dadurch gekennzeichnet, dass** das zumindest eine Nebenlumen (7) und das Hauptlumen (4) abgegrenzt und/oder separiert zueinander ausgebildet sind, sodass keine strömungsdurchlässige Verbindung zwischen Hauptlumen (4) und Nebenlumen (7) ausgebildet ist.

2. Kanüle (1) nach Anspruch 1, **dadurch gekennzeichnet, dass** das Nebenlumen (7) in der Kanülenwandung (2) ausgeformt ist.

3. Kanüle (1) nach Anspruch 1 oder 2, **dadurch gekennzeichnet, dass** der Kanülenkörper (3) eine an einer Innenmantelseite (13) und/oder einer Außenmantelseite (14) der Kanülenwandung (2) ausgeformte, das Nebenlumen (7) umgebende Sekundärleitung aufweist.

4. Kanüle (1) nach zumindest einem der vorhergehenden Ansprüche, **dadurch gekennzeichnet, dass** die Eintrittsöffnung (8) an der Stirnseite (10) der Kanülenwandung (2) positioniert ist, an welcher sich die Haupteinlassöffnung (5) befindet.

5. Kanüle (1) nach zumindest einem der vorhergehenden Ansprüche, **dadurch gekennzeichnet, dass** die Eintrittsöffnung (8) an einer Außenmantelseite (14) der Kanülenwandung (2) positioniert ist.

6. Kanüle (1) nach zumindest einem der vorhergehenden Ansprüche, **dadurch gekennzeichnet, dass** das Nebenlumen (7) wenigstens einen linearen Lumenabschnitt (11) und/oder wenigstens einen gekrümmten Lumenabschnitt (12) aufweist.

7. Kanüle (1) nach zumindest einem der vorhergehenden Ansprüche, **dadurch gekennzeichnet, dass** ein der Eintrittsöffnung (8) in Richtung der Austrittsöffnung (9) nacheilender Lumenabschnitt (11) des Nebenlumens (7) linear ausgebildet und zum Hauptlumen (4) oder einem Abschnitt des Hauptlumens (4) parallel ausgerichtet ist.

8. Kanüle (1) nach zumindest einem der vorhergehenden Ansprüche, **dadurch gekennzeichnet, dass** ein der Austrittsöffnung (9) in Richtung der Eintrittsöffnung (8) voreilender Lumenabschnitt (12) des Nebenlumens (7) gekrümmt ausgebildet ist und hierbei einem Bogen folgt, wobei eine in der Austrittsöffnung (9) an den Bogen angelegte Tangente parallel oder in einem spitzen Winkel zum Hauptlumen (4) ausgerichtet ist.

9. Kanüle (1) nach zumindest einem der vorhergehenden Ansprüche, **dadurch gekennzeichnet, dass** im Nebenlumen (7) eine Zweitkanüle angeordnet ist, welche sich über die Austrittsöffnung (9) oder die Eintrittsöffnung (8) und die Austrittsöffnung (9) hinaus erstreckt.

10. Kanüle (1) nach zumindest einem der vorhergehenden Ansprüche, **dadurch gekennzeichnet, dass** das Nebenlumen (7) als Führung für die Zweitkanüle ausgebildet und die Zweitkanüle gegenüber dem Kanülenkörper (3) verschiebbar im Nebenlumen (7) angeordnet ist.
